# EUROPÄISCHE PATENTSCHRIFT

(11) **EP 1 539 051 B1**
(45) Veröffentlichungstag und Bekanntmachung des Hinweises auf die Patenterteilung: **07.05.2008**
(21) Anmeldenummer: 02754125.9
(22) Anmeldetag: 18.09.2002
(51) Int. Cl.: A61F 2/44

(54) **IMPLANTAT MIT ZWEITEILIGEM GELENK**
IMPLANT COMPRISING A TWO-PIECE JOINT
IMPLANT COMPRENANT UNE ARTICULATION EN DEUX PARTIES

(43) Veröffentlichungstag der Anmeldung: 15.06.2005
(73) Patentinhaber: Synthes GmbH, 4436 Oberdorf (CH)
(72) Erfinder: BAUMGARTNER, Daniel, CH-4702 Oensingen (CH); BURRI, Adrian, CH-3900 Brig (CH); MATHIEU, Claude, 8002 Zurich (CH)
(74) Vertreter: Lusuardi, Werther
(86) Internationale Anmeldenummer: PCT/CH2002/000512
(87) Internationale Veröffentlichungsnummer: WO 2004/026186

(56) Entgegenhaltungen:
- WO-A-01/01893
- US-A- 5 405 400
- US-A- 5 645 605
- US-B1- 6 290 726
- US-B1- 6 368 350

## Beschreibung

Die Erfindung bezieht sich auf ein Implantat, insbesondere ein Zwischenwirbelimplantat gemäss dem Oberbegriff des Patentanspruchs 1.

Bei einzelnen Gelenken am menschlichen Körper, beispielsweise dem Gelenk zwischen distaler Femurkondyle und Patella, oder zwischen Metacarpalia und distaler Phalanx findet man Artikulationsflächen, welche eine bevorzugte Artikulation um eine oder mehrere Gelenkachsen zulassen und andererseits eine Artikulation um alle anderen räumlich möglichen Drehachsen einschränken oder gar nicht zulassen. Implantate mit einem mehrere Drehachsen aufweisenden Gelenk werden andererseits auch als Zwischenwirbelimplantate respektive Bandscheibenendoprothesen eingesetzt.

Eine oder mehrere beschädigte, natürliche Bandscheiben oder auch ein beschädigter Nukleus einer Bandscheibe werden üblicherweise entfernt und teilweise durch Implantate oder Prothesen ersetzt, welche in den Zwischenwirbelraum zwischen den beiden benachbarten Wirbelkörpern eingebracht werden. Dabei besteht das Ziel, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wieder herzustellen. Bewegungen der Wirbelkörper sollen ohne Beeinträchtigung durch das Implantat oder die Prothese auch weiterhin in ihrem natürlichen Bereich ausführbar sein. Hierzu ist die Erhaltung der Bewegungsmöglichkeiten bei einer Vorwärts/Rückwärtsneigung, d.h. Flexion oder Extension der Wirbelsäule sowie bei einer lateralen Beugung der Wirbelkörper innerhalb der natürlichen Grenzen wesentlich. Ebenfalls soll eine räumliche Positionsänderung der benachbarten Wirbelkörper relativ zueinander innerhalb des physiologischen Bewegungsbereiches ermöglicht werden.

Eine als Ersatz für eine Bandscheibe implantierbare Vorrichtung ist aus der US 6,368,350 ERICKSON bekannt. Diese bekannte Vorrichtung umfasst im wesentlichen zwei Gelenkteile mit Endplatten. Das aus einem konvexen und einem komplementär konkaven Gelenkteil bestehende Gelenk weist in einer Ausführungsform sphärische Artikulationsflächen auf, so dass Rotationen der Gelenkteile um verschiedene in einer Ebene liegende Drehachsen möglich sind und keine Einschränkung auf eine Drehachse für laterale Biegung der Wirbelkörper und eine weitere Drehachse für Flexion/Extension der Wirbelkörper stattfindet. Ferner ist eine Rotation der angrenzenden Wirbelkörper um deren Längsachse ohne Einschränkung möglich. In einer anderen Ausführungsform sind die Artikulationsflächen Oberflächen eines Ellipsoides, so dass die Drehachsen ebenfalls nicht auf eine Drehachse für laterale Biegung der Wirbelkörper und eine weitere Drehachse für Flexion/Extension der Wirbelkörper eingeschränkt werden.

Hier will die Erfindung Abhilfe schaffen. Der Erfindung liegt die Aufgabe zugrunde, ein Implantat mit zwei artikulierenden Gelenkstücken zu schaffen, welches zwei Drehachsen aufweist, die windschief zueinander stehen und einen definierten Abstand im Raum aufweisen, so dass die Bewegungen der angrenzenden Wirbelkörper nach Resektion der dazwischenliegenden Bandscheibe durch das Implantat reproduziert werden.

Die Erfindung löst die gestellte Aufgabe mit einem Implantat, vorzugsweise einem Zwischenwirbelimplantat, welches die Merkmale des Anspruchs 1 aufweist

Das erfindungsgemässe Implantat umfasst im wesentlichen zwei Gelenkstücke mit aufeinander artikulierenden Gleitflächen, welche sich bei einer Rotation der Gelenkstücke relativ zueinander aufeinander verschieben. Jedes der Gelenkstücke weist eine im wesentlichen parallel oder koaxial zu den Längsachsen von zwei angrenzenden Knochen verlaufende Zentralachse auf, welche in der Ruhestellung bei entsprechend aufrechter Körperhaltung, d.h. bei nicht gegeneinander abgekippten Gelenkstücken zusammenfallen. Ferner umfassen die Gelenkstücke je ein axial aussenstehendes, mit einem Knochen verbindbares Ende. Bei der Ausgestaltung des Implantates als Zwischenwirbelimplantat sind diese Enden mit den angrenzenden Wirbelkörpern verbindbar, während die Zentralachsen im wesentlichen parallel oder koaxial mit der Wirbelsäulenlängsachse verlaufen. Die Gleitflächen schneiden die Zentralachsen der Gelenkstücke und verschieben sich bei einer Rotation der Gelenkstücke relativ zueinander aufeinander, wobei das zweite Gelenkstück um zwei windschief zueinander angeordnete Drehachsen relativ zum ersten Gelenkstück rotierbar ist.

Im folgenden werden das erste Gelenkstück als feststehend und das zweite Gelenkstück als bewegbar angenommen. Damit sind die Drehachsen gegenüber dem zweiten Gelenkstück feststehend und gegenüber dem ersten Gelenkstück bewegbar.

Die durch die Erfindung erreichten Vorteile sind im wesentlichen darin zu sehen, dass dank des erfindungsgemässen Implantates, insbesondere Zwischenwirbelimplantates
- die Lage des Rotationszentrums bei Flexion oder Extension und/oder bei lateraler Biegung der Wirbelkörper besser an die Physiologie angepasst wird;
- die Gleitflächen ohne grosse Reibungskräfte aufeinander verschiebbar sind, und durch Reproduzieren der entsprechenden Hebelarme die Kraftverhältnisse und Momente möglichst minimiert werden; und
- eine Versteifung gegenüber Torsionsbewegungen der Wirbelkörper um die Wirbelsäulenlängsachse erreichbar ist.

In der bevorzugten Ausführungsform des erfindungsgemässen Implantates sind die Gleitflächen sattelförmig ausgestaltet und weisen je einen Sattelpunkt auf. Die sattelförmigen Gleitflächen sind derart ausgestaltet, dass sich in jeder der durch die Abmessungen der Gelenkteile begrenzten Umgebung eines Flächenpunktes P stets zwei Flächenpunkte angeben lassen, welche auf verschiedenen Seiten der Tangentialebene durch den Flächenpunkt P liegen.

In einer weiteren Ausführungsform des erfindungsgemässen Implantates kreuzen sich die Drehachsen unter einem Winkel, welcher vorzugsweise zwischen 80° und 100° beträgt. Dadurch ist der Vorteil erreichbar, dass beispielsweise ein Zwischenwirbelimplantat so implantierbar ist, dass eine der Drehachsen parallel oder koaxial zur Achse für die laterale Biegung Wirbelkörper und die zweite Drehachse parallel oder koaxial zur Achse für die Flexion oder Extension der Wirbelkörper ausgerichtet ist.

In einer anderen Ausführungsform des erfindungsgemässen Implantates weisen die Drehachsen einen minimalen Abstand A relativ zueinander auf, welcher zwischen 0,1 mm und 20 mm, vorzugsweise zwischen 2 mm und 20 mm beträgt. Der Abstand A wird auch durch den Einsatzort des erfindungsgemässen Implantates an der Wirbelsäule mitbestimmt und variiert je nach Segmenthöhe in der lumbalen Wirbelsäule und nimmt in Richtung der thorakalen Wirbelkörper ab.

Vorzugsweise sind die Gleitflächen derart ausgestaltet, dass sich bei einer Drehung des zweiten Gelenkstückes um jede der Drehachsen der zweite Sattelpunkt auf zu den Drehachsen konzentrischen Kreisbogen verschiebt. Die sattelförmige Ausgestaltung der Gleitflächen weist den Vorteil auf, dass auch eine Rotation der Gelenkstücke um die Zentralachsen der Gelenkstücke ermöglicht wird. Jedoch bewegen sich bei einer axialen Rotation der Gelenkstücke relativ zueinander die beiden Gelenkstücke axial voneinander weg, so dass eine axiale Rotation nur bei gleichzeitiger Höhenänderung des Implantates möglich ist. Durch die konstante Vorspannkraft der Bänder, Muskeln und Sehnen in der Wirbelsäule wird eine axiale Rotation der Gelenkstücke nur beschränkt möglich. Dies gilt analog zum physiologischen Fall in der lumbalen Wirbelsäule, wo die axiale Rotation aufgrund der posterioren Elemente, wie Facettengelenke auch nur beschränkt zugelassen wird.

Vorzugsweise sind die Gleitflächen im Ruhezustand der Gelenkstücke betrachtet kongruent ausgebildet. Durch die Ausgestaltung des erfindungsgemässen Implantates mit kongruenten Gleitflächen ist erreichbar, dass eine Torsionsbewegung der Wirbelkörper um die Wirbelsäulenlängsachse ohne Veränderung der Höhe des Zwischenwirbelimplantates nicht möglich ist. Durch eine Veränderung der Höhe des Zwischenwirbelimplantates bei einer solchen Bewegung werden die Bänder angespannt, wodurch ein Widerstand gegen Torsion der Wirbelkörper aufgebracht wird.

In einer weiteren Ausführungsform des erfindungsgemässen Implantates umfassen die aussenstehenden Enden der Gelenkstücke je ein Verbindungsteil, welches mit dem angrenzenden Knochen oder Wirbelkörper verbindbar ist. Bei der Anwendung des erfindungsgemässen Implantates als Zwischenwirbelimplantat sind diese Verbindungsteile vorzugsweise als Deckplatten mit je einer axial aussenstehenden, quer zu den Zentralachsen angeordneten Oberfläche ausgestaltet, wobei diese Oberflächen mit einer dreidimensionalen Strukturierung, beispielsweise mit Finnen ausgestattet sein können.

Dabei kann auch eine der Deckplatten mit dem angrenzenden Gelenkstück einstückig sein.

In einer Ausführungsform des erfindungsgemässen Implantates als Zwischenwirbelimplantat umfasst eine der Deckplatten eine senkrecht zur Zentralachse des angrenzenden Gelenkstückes verlaufende Führung oder Nute, in welche das komplementär zu dieser Führung ausgestaltete, hintere Ende des angrenzenden Gelenkstückes einschiebbar ist. Damit ist der Vorteil erreichbar, dass zuerst die beiden Deckplatten mit dem ersten Gelenkstück aneinanderliegend zwischen die angrenzenden Wirbelkörper geschoben werden können, anschliessend die Deckplatten an die Endflächen der angrenzenden Wirbelkörper gepresst werden können und erst zuletzt das zweite Gelenkstück zwischen das erste Gelenkstück und die zweite Deckplatte eingefügt wird, so dass die Wirbelkörper während der Implantation nur minimal distrahiert werden müssen. Das zweite eingefügte Gelenkstück wird nach dem Einführen an die zweite Deckplatte fixiert.

Die Gelenkstücke können je nach Anwendung als Metall/Metall- Paarung ausgestaltet sein. Ferner sind auch Anwendungen von Kermamikmaterialien geeignet, da wegen der hohen Vorspannkräfte zwischen den angrenzenden Wirbelkörpern in der Wirbelsäule die Belastung der Gelenkstücke auf Schockeinwirkungen gering ist.

In einer weiteren Ausführungsform des erfindungsgemässen Implantates ist eines der Gelenkstücke aus einem Kunststoff hergestellt, so dass
- bereits bewährte Kombinationen von Gelenkersatzmaterialien wie beispielsweise ein hochvernetztes Polyethylen (UHMWPE) und eine Kobalt-Chrom Legierung einsetzbar sind;
- geringe Reibungskräfte bei der Verschiebung der Gleitflächen relativ zueinander erreichbar sind; und
- eine Dämpfung für die Rotationsbewegung der Gelenkstücke um die Zentralachsen herstellbar ist.

In wiederum einer weiteren Ausführungsform des erfindungsgemässen Implantates ist eines der Gelenkstücke um seine Zentralachse rotierbar am zugehörigen Verbindungsteil, respektive an der zugehörigen Deckplatte aufnehmbar. Dazu kann beispielsweise das aussenstehende Ende des Gelenkstückes in einer komplementären, zur Zentralachse koaxialen Vertiefung am zugehörigen Verbindungsteil, respektive der zugehörigen Deckplatte gelagert sein. Andererseits kann das Verbindungsteil mit einer zur Zentralachse koaxialen Erhebung und das Gelenkstück mit einer komplementären Vertiefung versehen sein. Damit ist der Vorteil erreichbar, dass Torsionsbewegungen der beiden angrenzenden Wirbelkörper durch das Implantat nicht verhindert werden.

In einer anderen Ausführungsform des erfindungsgemässen Implantates ist eines der Gelenkstücke parallel zu einer senkrecht zu Zentralachse verlaufenden Verschiebeachse verschiebbar am zugehörigen Verbindungsteil, respektive der zugehörigen Deckplatte aufnehmbar. Vorzugsweise ist das aussenstehende Ende des Gelenkstückes endständig mit einer zur Zentralachse koaxialen Erweiterung versehen, während das zugehörige Verbindungsteil, respektive die zugehörige Deckplatte eine zum aussenstehenden Ende des Gelenkstückes komplementäre Vertiefung mit einem Hinterstich zu Aufnahme der Erweiterung umfasst. Durch diese Ausgestaltung des erfindungsgemässen Implantates sind auch einachsige Scherbewegungen zwischen den beiden an das Implantat angrenzenden Wirbelkörper möglich, ohne dass diese durch das Implantat verhindert werden. Durch die Ausgestaltung der Länge der Vertiefung kann die Scherbewegung der Wirbelkörper innerhalb der gewünschten Begrenzung eingeschränkt werden.

In wiederum einer anderen Ausführungsform des erfindungsgemässen Implantates ist eines der Gelenkstücke in einer zur Zentralachse senkrecht stehenden Ebene verschiebbar am zugehörigen Verbindungsteil, respektive der zugehörigen Deckplatte aufnehmbar. Vorzugsweise weist hierzu dass aussenstehende Ende des Gelenkstückes einen kleineren Durchmesser auf als die Vertiefung an dem zugehörigen Verbindungsteil, respektive der zugehörigen Deckplatte. Dadurch ist der Vorteil erreichbar, dass auch Scherbewegungen der an das Implantat angrenzenden Wirbelkörper bezüglich mehrerer Achsen zugelassen werden können.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1a eine Ansicht einer Ausführungsform des erfindungsgemässen Implantates, wobei das zweite Gelenkstück um die erste Drehachse rotiert ist;
Fig. 1b eine Seitenansicht der in Fig. 1a dargestellten Ausführungsform des erfindungsgemässen Implantates;
Fig. 1c eine Ansicht der in den Fig. 1a und 1b dargestellten Ausführungsform des erfindungsgemässen Implantates, wobei das zweite Gelenkstück um die zweite Drehachse rotiert ist;
Fig. 1d eine Seitenansicht der in Fig. 1c dargestellten Ausführungsform des erfindungsgemässen Implantates;
Fig. 2 eine perspektivische Ansicht des ersten Gelenkstückes einer Ausführungsform des erfindungsgemässen Implantates;
Fig. 3 eine perspektivische Ansicht einer Ausführungsform des erfindungsgemässen Implantates als Zwischenwirbelimplantat;
Fig. 4a eine Ansicht von ventral auf eine Ausführungsform des erfindungsgemässen Implantates;
Fig. 4b eine Ansicht von lateral auf die in Fig. 4a dargestellte Ausführungsform des erfindungsgemässen Implantates.
Fig. 5 eine Ansicht einer weiteren Ausführungsform des erfindungsgemässen Implantates mit relativ zur Zentralachse rotierbar an der Deckplatte angeordnetem Gelenkstück;
Fig. 6a ein Ansicht einer anderen Ausführungsform des erfindungsgemässen Implantates mit in einer zur Zentralachse senkrecht stehenden Ebene verschiebbar angeordnetem Gelenkstück;
Fig. 6b einen Schnitt durch die in Fig. 6a dargestellte Ausführungsform des erfindungsgemässen Implantates;
Fig. 7a eine Ansicht einer weiteren Ausführungsform des erfindungsgemässen Implantates mit senkrecht zur Zentralachse verschiebbar angeordnetem Gelenkstück; und
Fig. 7b einen Schnitt durch die in Fig. 7a dargestellte Ausführungsform des erfindungsgemässen Implantates.

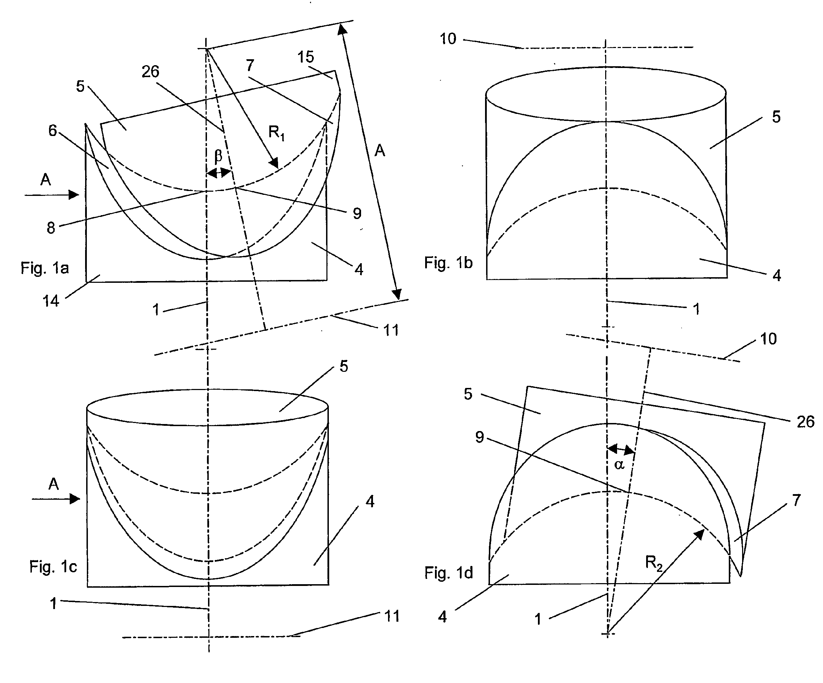

In den Fig. 1a bis 1d ist eine Ausführungsform des erfindungsgemässen Implantates mit einem ersten und einem zweiten Gelenkstück 4;5 dargestellt, wobei in Fig. 1b die beiden Gelenkstücke 4;5 von der Seitenansicht A gezeigt werden. Das erste Gelenkstück 4 weist eine erste Zentralachse 1 und eine erste, diese erste Zentralachse 1 schneidende Gleitfläche 6 auf. Diese erste Gleitfläche 6 ist sattelförmig ausgestaltet und hat einen ersten Sattelpunkt 8. Analog zum ersten Gelenkstück 4 weist das zweite Gelenkstück 5 eine zweite Zentralachse 26 und eine zweite, diese zweite Zentralachse 26 schneidende Gleitfläche 7 auf. Auch die zweite Gleitfläche 7 ist sattelförmig ausgestaltet und weist den Sattelpunkt 9 auf. Ferner umfassen die Gelenkstücke 4;5 aussenstehende Enden 14;15, welche an den Endflächen von an die Gelenkstücke angrenzenden Knochen, insbesondere von angrenzenden Wirbelkörpern zur Anlage bringbar sind. In einer zur Zentralachse 1 orthogonalen Ebene betrachtet schneiden sich die Projektionen der Drehachsen 10;11 in diese Ebene unter einem Winkel von 90°. Der minimale Abstand A ist bei dieser Ausgestaltung der Gleitlächen 6;7 die Senkrechte auf den beiden Drehachsen 10;11. In den Fig. 1a und 1b ist das zweite Gelenkstück 5 um die erste Drehachse 10 (Fig. 1 b) rotiert. Der zweite Sattelpunkt 9 wird bei der Rotationsbewegung des zweiten Gelenkstückes 5 um den Winkel β auf einem zur ersten Drehachse 10 konzentrischen Kreisbogen mit dem Radius R₁ verschoben.

Die Fig. 1c und 1d zeigen die Gelenkstücke 4;5 der in den Fig. 1a und 1b dargestellten Ausführungsform des erfindungsgemässen implantates, wobei das zweite Gelenkstück 5 um die zweite Drehachse 11 (Fig. 1 c) um den Winkel α rotiert ist. Bei dieser Rotation des zweiten Gelenkstückes 5 um die zweite Drehachse 11 wird der zweite Sattelpunkt 9 auf einem zur zweiten Drehachse 11 konzentrischen Kreisbogen mit dem Radius R₂ verschoben.

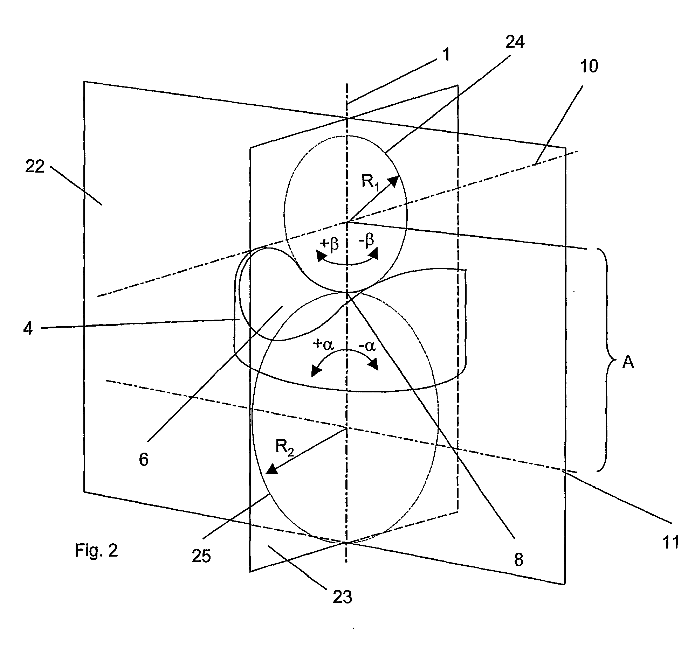

In Fig. 2 ist das erste Gelenkstück 4 mit einer sattelförmig ausgestalteten ersten Gleitfläche 6 dargestellt. Die beiden Drehachsen 10;11 sind in der Ruhelage des erfindungsgemässen Implantates bei entsprechend aufrechter Körperhaltung dargestellt, d.h. die Zentralachse 1 des ersten Gelenkstückes 4 fällt mit der Zentralachse 26 des zweiten Gelenkstückes 5 (Fig. 1) zusammen. In der Ruhelage stehen die Drehachsen 10;11 senkrecht auf der Zentralachse 1 und liegen in Ebenen 22;23. Die erste Drehachse 10 steht senkrecht auf einer ersten Ebene 22, welche durch die Zentralachse 1 und die zweite Drehachse 11 aufgespannt wird. Die zweite Drehachse 11 steht senkrecht auf einer zweiten, zur ersten Ebene 22 senkrecht stehenden Ebene 23, welche durch die Zentralachse 1 und die erste Drehachse 10 aufgespannt wird. Der erste Sattelpunkt 8 der ersten Gleitfläche 6 liegt in der Ruhelage sowohl auf der Zentralachse 1 wie auch auf zwei Kreisbogen 24;25, deren Zentrum in der Ruhelage der Gelenkstücke 4;5 durch den Schnittpunkt der Zentralachse 1 mit je einer der Drehachsen 10;11 definiert ist. Die erste Gleitfläche 6 schmiegt sich einerseits an den ersten, zur ersten Drehachse 10 konzentrischen Kreisbogen 24 und andererseits auch an den zweiten, zur zweiten Drehachse 11 konzentrischen Kreisbogen 25. Die Radien R₁ des ersten Kreisbogens 24 und R₂ des zweiten Kreisbogens 25 sind bei kongruenten Gleitflächen 7;8 gleich gross und entsprechen dem halben Abstand A der beiden Drehachsen 10;11.

Die Gleitfläche 6 ist derart ausgestaltet, dass sie die komplementäre Form eines Ausschnittes aus einer Torusoberfläche darstellt.

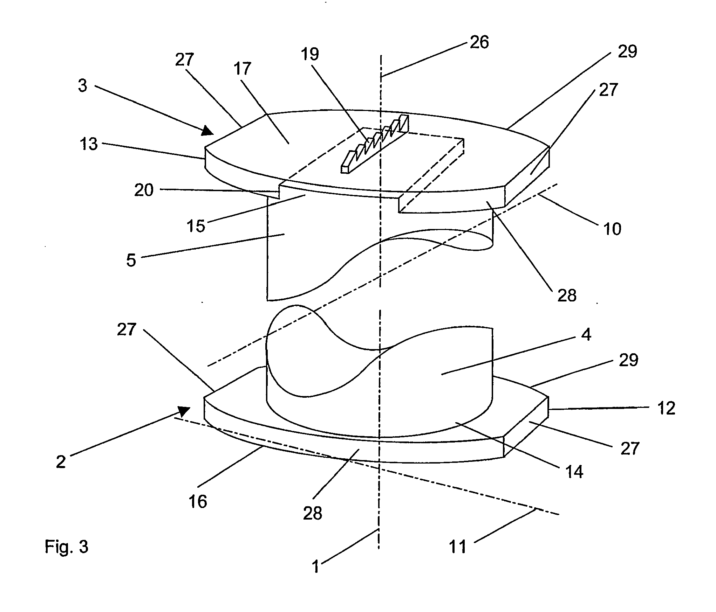

In Fig. 3 ist eine Ausführungsform des erfindungsgemässen Implantates als Zwischenwirbelimplantat dargestellt. Die beiden Gelenkstücke 4;5 umfassen an ihren aussenstehenden Enden 14;15 als Verbindungsteile 2;3 je eine Deckplatte 12;13, mit bezüglich den Zentralachsen 1;26 axial aussenstehenden und quer zu den Zentralachsen 1;26 stehenden Oberflächen 16;17, welche an die Endflächen der angrenzenden Wirbelkörper zur Anlage bringbar sind. Die Deckplatten 12;13 weisen je zwei laterale Seitenflächen 27, eine anteriore Seitenfläche 28 und eine posteriore Seitenfläche 29 auf, wobei die lateralen Seitenfläche 27 im wesentlichen parallel zur ersten Drehachse 10 angeordnet sind. Die anteriore sowie die posteriore Seitenfläche 28;29 sind im wesentlichen parallel zur zweiten Drehachse 11 angeordnet. Das Implantat wird so zwischen die angrenzenden Wirbelkörper (nicht gezeichnet) eingeführt, dass eine Rotation der Gelenkstücke 4;5 um die erste Drehachse 10 eine laterale Biegung der mit dem Implantat verbundenen Wirbelkörper ermöglicht, während eine Rotation der Gelenkstücke 4;5 um die zweite Drehachse 11 eine Flexion respektive Extension der mit dem Implantat verbundenen Wirbelkörper gestattet. In der hier dargestellten Ausführungsform des erfindungsgemässen Implantates ist die erste Deckplatte 12 mit dem ersten Gelenkstück 4 fest verbunden, während die zweite Deckplatte 13 eine senkrecht zur Zentralachse 26 stehende und im wesentlichen parallel zur den lateralen Seitenflächen 27 angeordnete, als Nute ausgebildete Führung 20 aufweist, so dass das zweite Gelenkstück 5 mit seinem aussenstehenden, zur Führung 20 komplementär ausgebildeten Ende 15 in die Führung 20 einschiebbar ist. Ferner sind auf den aussenstehenden Oberflächen 16;17 der Deckplatten 12;13 Finnen 19 angebracht, welche zur primären Stabilisation des lmplantates an den angrenzenden Wirbel körpern dienen.

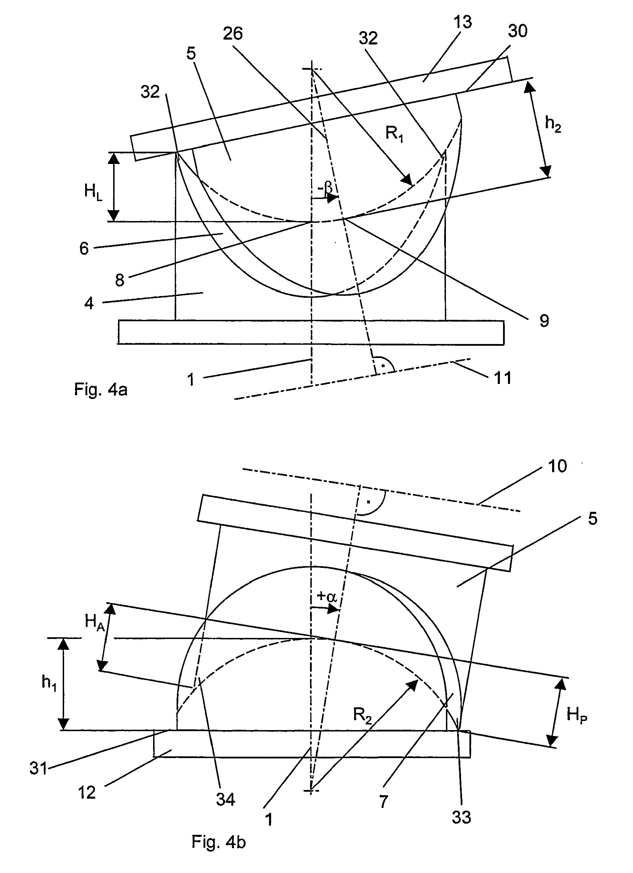

Die in den Fig. 4a und 4b dargestellte Ausführungsform des erfindungsgemässen Implantates unterscheidet sich von der in Fig. 3 gezeigten Ausführungsform des erfindungsgemässen Implantates nur darin, dass sie keine Finnen 19 umfasst und dass die Gelenkstücke 4;5 nur innerhalb begrenzter Drehwinkel α und β um die Drehachsen 10;11 rotierbar sind. In Fig. 4a ist das Implantat von ventral, d.h. parallel zur Drehachse 10 betrachtet gezeigt und in Fig. 4b ist das Implantat von lateral, d.h. parallel zur Drehachse 11 betrachtet dargestellt (Fig. 4b). Die Begrenzung der Drehwinkel α und β wird durch die Wahl der Abmessungen H_{L}; H_{A}; H_{P}; R₁; R₂; h₁ und h₂ an den Gelenkstücken 4; 5 hergestellt, wobei bei Erreichen des gewünschten maximalen Drehwinkels α oder β die jeweils gegen das andere Gelenkstück 4;5 gerichteten Enden 32; 33; 34 der Gelenkstücke 4;5 auf den Innenflächen 30;31 der dem jeweiligen Gelenkstück 4;5 gegenüberliegenden Deckplatte 12;13 anstehen und
H_{L}: die Höhe zwischen dem ersten Sattelpunkt 8 und den inneren, gegen das zweite Gelenkstück 5 gerichteten Enden 32 des ersten Gelenkstückes 4 ist;
H_{A}: die Höhe zwischen dem zweiten Sattelpunkt 9 und dem anterioren, gegen das erste Gelenkstück 4 gerichteten Ende 34 des zweiten Gelenkstückes 5 ist;
Hp: die Höhe zwischen dem zweiten Sattelpunkt 9 und dem posterioren, gegen das erste Gelenkstück 4 gerichteten Ende 33 des zweiten Gelenkstückes 5 ist;
R₁: der Radius der ersten Gleitfläche 6 in der ersten, zur ersten Drehachse 10 senkrechten und den ersten Sattelpunkt 8 enthaltenden Ebene 22 (Fig. 2) ist;
R₂: der Radius der zweiten Gleitfläche in der zweiten, zur zweiten Drehachse 11 senkrechten und den zweiten Sattelpunkt 9 enthaltenden Ebene 23 (Fig. 2) ist;
h₁: die Höhe zwischen dem ersten Sattelpunkt 8 und der Innenfläche 31 der ersten Deckplatte 12 ist; und
h₂: die Höhe zwischen dem zweiten Sattelpunkt 9 und der Innenfläche 30 der zweiten Deckplatte 13 ist.

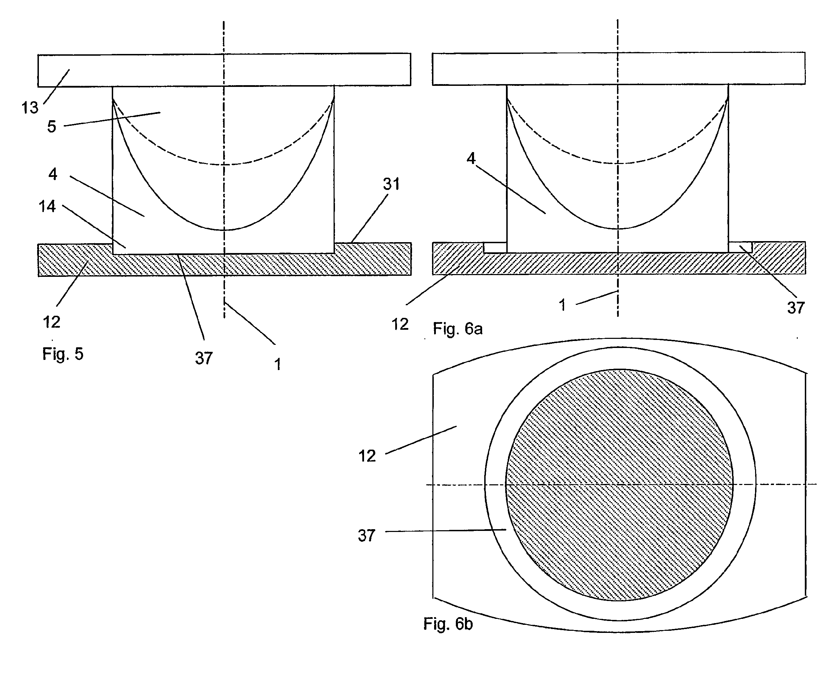

In Fig. 5 ist eine Ausführungsform des erfindungsgemässen Implantates dargestellt, welche sich von den in den Fig. 1 bis 4 dargestellten Ausführungsformen darin unterscheidet, dass das aussenstehende Ende 14 des ersten Gelenkstückes 4 in einer komplementären, zur Zentralachse 1 koaxialen Vertiefung 37 in der Deckplatte 12 aufgenommen wird, so dass das erste Gelenkstück 4 um die Zentralachse 1 rotierbar mit der Deckplatte 12 zusammenfügbar ist.

In den Fig. 6a und 6b ist eine Ausführungsform des erfindungsgemässen Implantates dargestellt, welche sich von der in Fig. 5 dargestellten Ausführungsformen nur darin unterscheidet, dass die Vertiefung 37 zur Zentralachse 1 einen grösseren Durchmesser aufweist als das aussenstehende Ende 14 des ersten Gelenkstückes 4, so dass das erste Gelenkstück 4 in einer zur Zentralachse 1 senkrecht stehenden Ebene relativ zur Deckplatte 12 verschiebbar ist.

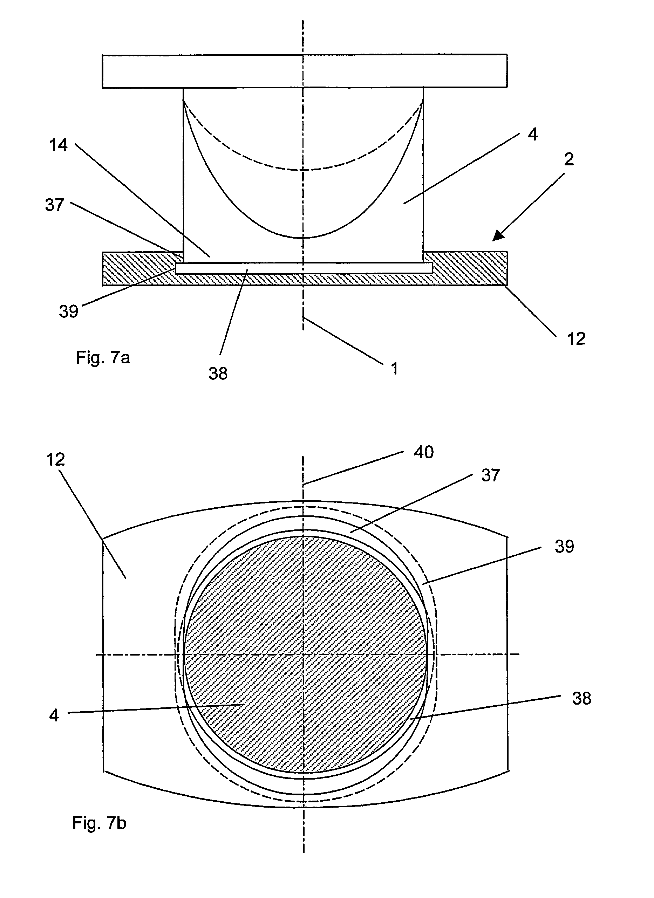

In den Fig. 7a und 7b ist eine Ausführungsform des erfindungsgemässen implantates dargestellt, welche sich von der in Fig. 5 dargestellten Ausführungsform nur darin unterscheidet, dass die Vertiefung 37 parallel zu einer senkrecht zur Zentralachse 1 stehenden Verschiebeachse 40 oval ausgestaltet ist und einen Hinterstich 39 aufweist, während das aussenstehende Ende 14 des ersten Gelenkstückes 4 endständig eine zur Zentralachse 1 koaxiale Erweiterung 38 umfasst, welche in den Hinterstich 39 eingreift, so dass das erste Gelenkstück 4 einerseits parallel zur Verschiebeachse 40 verschiebbar und andererseits durch die in den Hinterstich 39 eingreifende Erweiterung 38 bezüglich der Zentralachse 1 axial gesichert ist.

## Patentansprüche

1. implantat, insbesondere Zwischenwirbelimplantat mit
A) zwei Gelenkstücken (4;5), welche je eine Zentralachse (1;26), je eine die Zentralachsen (1;26) schneidende Gleitfläche (6;7) und je ein axial aussenstehendes, mit einem Knochen verbindbares Ende (14;15) aufweisen, wobei
B) die Gleitflächen (6;7) gekrümmt ausgebildet sind,
C) die Gleitflächen aufeinander verschiebbar sind; und
D) das zweite Gelenkstück (5) um zwei windschief angeordnete Drehachsen (10;11) relativ zum ersten Gelenkstück (4) rotierbar ist,
E) die aussenstehenden Enden (14;15) der Gelenkstücke (4;5) je ein Verbindungsteil (2;3) umfassen; wobei
F) ein Verbindungsteil (2;3) eine zur Zentralachse (1 ;26) koaxiale, ovale Vertiefung (37) zur Aufnahme des aussenstehenden Endes (14;15) des angrenzenden Gelenkstückes (4;5) aufweist;
**dadurch gekennzeichnet, dass**
G) die Vertiefung (37) axial endständig einen Hinterstich (39) aufweist, und dass das aussenstehende Ende (14;15) des angrenzenden Gelenkstückes (4;5) eine zur Zentralachse (1;26) koaxiale Erweiterung (38) aufweist, welche im Hinterstich (39) aufnehmbar ist; und
H) die Gleitflächen (6;7) sattelförmig ausgestaltet sind.

2. Implantat nach Anspruch 1, **dadurch gekennzeichnet, dass** die Gleitflächen (6;7) je einen Sattelpunkt aufweisen.

3. Implantat nach Anspruch 1 oder 2, **dadurch gekennzeichnet, dass** die Drehachsen (10;11) sich unter einem Winkel zwischen 80° und 100° kreuzen.

4. Implantat nach einem der Ansprüche 1 bis 3, **dadurch gekennzeichnet, dass** die Drehachsen (10;11) einen minimalen Abstand A relativ zueinander aufweisen und dass dieser Abstand A zwischen 0,1 mm und 20 mm beträgt.

5. Implantat nach Anspruch 4, **dadurch gekennzeichnet, dass** der Abstand A zwischen 2 mm und 20 mm beträgt.

6. Implantat nach einem der Ansprüche 1 bis 5, **dadurch gekennzeichnet, dass** die Gleitflächen (6;7) je einen Sattelpunkt (8;9) aufweisen, wobei sich bei einer Drehung des zweiten Gelenkstückes (5) um jede der Drehachsen (10;11) der zweite Sattelpunkt (9) auf einem zu der jeweiligen Drehachse (10;11) konzentrischen Kreisbogen (24;25) verschiebt.

7. implantat nach einem der Ansprüche 1 bis 6, **dadurch gekennzeichnet, dass** die Gleitflächen (6;7) in der Ausgangsstellung bei koaxialen Zentralachsen (1 ;26) der Gelenkstücke (4;5) kongruent ausgestaltet sind.

8. Implantat nach einem der Ansprüche 1 bis 7, **dadurch gekennzeichnet, dass** die Verbindungsteile (2;3) als Deckplatten (12;13) mit je einer axial aussenstehenden, quer zu den Zentralachsen (1;26) angeordneten Oberfläche (16;17) ausgestaltet sind.

9. Implantat nach Anspruch 8, **dadurch gekennzeichnet, dass** eine der Deckplatten (12;13) mit dem angrenzenden Gelenkstück (5) einstückig ist.

10. Implantat nach Anspruch 8 oder 9, **dadurch gekennzeichnet, dass** eine der Deckplatten (12) eine senkrecht zur Zentralachse (1) verlaufende Führung (20) umfasst, und dass das angrenzende Gelenkstück (4) ein hinteres Ende (14) aufweist, welches in die Führung (20) einschiebbar ist.

11. Implantat nach einem der Ansprüche 1 bis 10, **dadurch gekennzeichnet, dass** eines der Gelenkstücke (4;5) um dessen Zentralachse (1;26) rotierbar mit dem zugehörigen Verbindungsteil (2;3) zusammenfügbar ist.

12. Implantat nach einem der Ansprüche 1 bis 11, **dadurch gekennzeichnet, dass** eines der Gelenkstücke (4;5) auf einer senkrecht zu dessen Zentralachse (1;26) stehenden Verschiebeachse (40) verschiebbar mit dem zugehörigen Verbindungsteil (2;3) zusammenfügbar ist.

13. Implantat nach einem der Ansprüche 1 bis 12, **dadurch gekennzeichnet, dass** eines der Gelenkstücke (4;5) in einer senkrecht zu dessen Zentralachse (1;26) stehenden Ebene verschiebbar mit dem zugehörigen Verbindungsteil (2;3) zusammenfügbar ist.

14. Implantat nach einem der Ansprüche 1 bis 13, **dadurch gekennzeichnet, dass** eines der Gelenkstücke (4;5) aus einem Kunststoff hergestellt ist.

15. Implantat nach einem der Ansprüche 1 bis 14, **dadurch gekennzeichnet, dass** mindestens eines der Gelenkstücke (4;5) aus einem Keramikmaterial besteht.

## Claims

1. An implant, in particular an intervertebral implant, comprising:
(A) two articulating parts (4; 5) each having a central axis (1; 26), each having a slide surface (6; 7) intersecting the central axes (1; 26) and each having an outermost end (14; 15) which can be connected to a bone, where
(B) the slide surfaces (6; 7) are curved,
(C) the slide surfaces are mutually displaceable, and
(D) the second slide surface (5) is rotatable about two skewed axes of rotation (10; 11) relative to the first articulating part (4),
(E) the outermost ends (14; 15) of the articulating parts each comprise a connection element (2; 3), where
(F) a connecting element (2; 3) is fitted with an oval recess (37) coaxial with the central axis (1; 26) to receive the outermost end (14; 15) of the adjoining articulating part (4; 5)
**characterized in that**
(G) the recess (37) is fitted with an axially terminal cavity (39) and **in that** the outermost end (14; 15) of the adjacent articulating part (4; 5) comprises a widening (38) coaxial with the central axis (1; 26), said widening being insertable into the cavity (39), and
(H) the slide surfaces (6; 7) are saddle-shaped.

2. Implant as claimed in claim 1, **characterized in that** the slide surfaces (6; 7) each comprise a saddle point.

3. Implant as claimed in either of claims 1 and 2, **characterized in that** the axes of rotation (10; 11) cross each other at an angle between 80 and 100°.

4. Implant as claimed in one of claims 1 through 3, **characterized in that** the axes of rotation (10; 11) are apart a minimum distance A that is between 0,1 and 20 mm.

5. Implant as claimed in claim 4, **characterized in that** the distance A is between 2 and 20 mm.

6. Implant as claimed in one of claims 1 through 6, **characterized in** the slide surfaces (6; 7) each comprise a saddle-point (8; 9) where, when the second articulating part (5) is rotated about each of the axes of rotation (10; 11), the second saddle point (9) moves along an arc of circle (24; 25) concentric with the particular axis of rotation (10; 11).

7. Implant as claimed in one of claims 1 through 6, **characterized in that**, in the initial position, the slide surfaces (6; 7) are congruent at coaxial central axes (1; 26) of the articulating parts (4; 5).

8. Implant as claimed in one of claims 1 through 7, **characterized in that** the connection elements (2; 3) are designed as cover plates (12; 13) each with an axially outermost surface (16; 17) transverse to the central axes (1; 26).

9. Implant as claimed in claim 8, **characterized in that** one of the cover plates (12; 13) is integral with the adjoining articulating part (5).

10. Implant as claimed in either of claims 8 and 9, **characterized** one of the cover plates (12) is fitted with a guide (20) perpendicular to the central axis (1) and in that the adjoining articulating part (4) comprises a rear end (14) insertable into the guide (20).

11. Implant as claimed in one of claims 1 through 10, **characterized in that** one of the articulating parts (4; 5) can be assembled to the associated connection element (2; 3) in a manner rotatably about its central axis (1; 26).

12. Implant as claimed in one of claims 1 through 11, **characterized in that** one of the articulating parts (4; 5) can be assembled to the associated connection element (2; 3) in a manner displaceably in a plane perpendicular to its central axis (1; 26).

13. Implant as claimed in one of claims 1 througfh 12, **characterized in that** one of the articulating parts (4; 5) can be assembled to the associated connection element (2; 3) in a manner displaceably in a plane perpendicular to its central axis (1; 26).

14. Implant as claimed in one of claims 1 through 13, **characterized in that** one of the articulating parts (4; 5) is made of plastic.

15. Implant as claimed in one of claims 1 through 14, **characterized in that** at least one of the articulating parts (4; 5) is made of a ceramic.

## Revendications

1. Implant, en particulier implant intervertébral comprenant
A) deux parties d'articulation (4 ; 5), lesquelles présentent chacune un axe central (1 ; 26), une surface de glissement (6 ; 7) coupant les axes centraux (1 ; 26) et une extremité axialement externe (14 ; 15) pouvant être reliée à un os, dans lequel
B) les surfaces de glissement (6 ; 7) sont conçues sous une forme courbée,
C) les surfaces de glissement peuvent être déplacées l'une sur l'autre ; et
D) la deuxième partie d'articulation (5) peut tourner par rapport à la première partie d'articulation (4), autour de deux axes de rotation (10 ; 11) disposés de manière gauche ;
E) les extrémités externes (14; 15) des parties d'articulation (4 ; 5) comprennent chacune un élément de liaison (2 ; 3) ; implant dans lequel
F) un élément de liaison (2 ; 3) présente un creux (37) ovale, coaxial à l'axe central (1 ; 26), pour loger l'extremité externe (14 ; 15) de la partie d'articulation adjacente (4 ; 5) ;
**caractérisé en ce que**
G) le creux (37) présente une rainure (39) axialement terminale, et **en ce que** l'extrémité externe (14 ; 15) de la partie d'articulation (4 ; 5) adjacente présente une extension (38) coaxiale à l'axe central (1 ; 26), laquelle peut être logée dans la rainure (39) ; et
H) les surfaces de glissement (6 ; 7) sont conçues en forme de selle.

2. Implant selon la revendication 1, **caractérisé en ce que** les surfaces de glissement (6 ; 7) présentent chacune un point selle.

3. Implant selon la revendication 1 ou 2, **caractérisé en ce que** les axes de rotation (10 ; 11) se croisent en formant un angle compris entre 80° et 100°.

4. Implant selon l'une quelconque des revendications 1 à 3, **caractérisé en ce que** les axes de rotation (10 ; 11) présentent une distance minimale A l'un par rapport à l'autre et **en ce que** cette distance A est comprise entre 0,1 mm et 20 mm.

5. Implant selon la revendication 4, **caractérisé en ce que** la distance A est comprise entre 2 mm et 20 mm.

6. Implant selon l'une quelconque des revendications 1 à 5, **caractérisé en ce que** les surfaces de glissement (6 ; 7) présentent chacune un point selle (8 ; 9), le deuxième point selle (9) se déplaçant, lors d'une rotation de la deuxième partie d'articulation (5) autour de chacun des axes de rotation (10 ; 11), sur un arc de cercle (24 ; 25) concentrique aux axes de rotation (10 ; 11) respectifs.

7. Implant selon l'une quelconque des revendications 1 à 6, **caractérisé en ce que** les surfaces de glissement (6 ; 7) sont conçues de manière congruente dans la position de départ lorsque les axes centraux (1 ; 26) des parties d'articulation (4 ; 5) sont coaxiaux.

8. Implant selon l'une quelconque des revendications 1 à 7, **caractérisé en ce que** les éléments de liaison (2 ; 3) sont conçus sous forme de plaques de recouvrement (12 ; 13) ayant chacune une surface (16 ; 17) axialement externe, disposée transversalement aux axes centraux (1 ; 26).

9. Implant selon la revendication 8, **caractérisé en ce que** l'une des plaques de recouvrement (12 ; 13) est solidaire de la partie d'articulation (5) adjacente.

10. Implant selon la revendication 8 ou 9, **caractérisé en ce que** l'une des plaques de recouvrement (12) comprend un guidage (20) s'étendant perpendiculairement à l'axe central (1), et **en ce que** la partie d'articulation (4) adjacente présente une extrémité arrière (14), laquelle peut être insérée dans le guidage (20).

11. Implant selon l'une quelconque des revendications 1 à 10, **caractérisé en ce que** l'une des parties d'articulation (4 ; 5) peut être assemblée avec l'élément de liaison associé (2 ; 3) de manière à pouvoir tourner autour de son axe central (1 ; 26).

12. Implant selon l'une quelconque des revendications 1 à 11, **caractérisé en ce que** l'une des parties d'articulation (4 ; 5) peut être assemblée avec l'élément de liaison associé (2 ; 3) de manière à pouvoir être déplacée selon un axe de déplacement (40) perpendiculaire à son axe central (1 ; 26).

13. Implant selon l'une quelconque des revendications 1 à 12, **caractérisé en ce que** l'une des parties d'articulation (4 ; 5) peut être assemblée avec l'élément de liaison associé (2 ; 3) de manière à pouvoir être déplacée dans un plan perpendiculaire à son axe central (1 ; 26).

14. Implant selon l'une quelconque des revendications 1 à 13, **caractérisé en ce que** l'une des parties d'articulation (4 ; 5) est faite de matière plastique.

15. Implant selon l'une quelconque des revendications 1 à 14, **caractérisé en ce qu'**au moins l'une des parties d'articulation (4 ; 5) est faite de céramique.
